# EUROPEAN PATENT APPLICATION

(11) **EP 4 316 486 A1**
(43) Date of publication of application: **07.02.2024**
(21) Application number: 22781424.1
(22) Date of filing: 10.03.2022
(51) Int. Cl.: A61K 31/357, A61P 21/00, C07D 317/72

(54) **NOVEL USE OF 3-(4-(BENZYLOXY)PHENYL)HEX-4-INOIC ACID DERIVATIVE**

(30) Priority: 01.04.2021 KR 20210042939
(71) Applicant: Hyundai Pharm Co., Ltd., Cheonan-si, Chungcheongnam-do 31213 (KR)
(72) Inventor: YOO, Hye Jin, Seoul 06599 (KR); HWANG, Hwan Jin, Ansan-si Gyeonggi-do 15336 (KR)
(74) Representative: Vossius & Partner Patentanwälte Rechtsanwälte mbB
(86) International application number: PCT/KR2022/003384
(87) International publication number: WO 2022/211303

(57) **Abstract**

A 3-(4-(benzyloxy)phenyl)hex-4-inoic acid derivative according to the present invention promotes the expression of the myogenic factors MyoG and MyHC while inhibiting the expression of the muscle atrophy factor atrogin-1, promotes the formation of myotubes, and inhibits myocyte apoptosis, and thus can promote myogenesis and inhibit muscle atrophy.

## Description

### [Technical Field]

The present invention relates to a novel use of a 3-(4-(benzyloxy)phenyl)hex-4-inoic acid derivative. Particularly, the present invention relates a use of a 3-(4-(benzyloxy)phenyl)hex-4-inoic acid derivative for the prevention or treatment of muscle diseases.

### [Background Art]

The United Nations (UN) defines people aged 65 or older as "elderly," and classifies a society in which the elderly population accounts for 7% or more of the total population as an `aging society,' a society in which the elderly population accounts for 14% or more of the total population as an `aged society,' and a society in which the elderly population accounts for 20% or more of the total population as a 'super-aged society.' Korea became an aging society in 2000, as the elderly population accounted for 7.2% of the total population, and became an aged society, as the elderly population aged 65 or older accounted for 15.7% in 2020 (Statistics of the elderly in 2020, National Statistical office).

As the elderly population increases, research and interest in geriatric diseases are increasing. A person's muscle mass decreases with age, decreasing 10 to 15% by the age of 50 to 70, and 30% or more by the age of 70 to 80. As a result, muscle strength and muscle function weaken, and muscle loss due to aging is called age-related sarcopenia. Age-related sarcopenia is a major cause that limits the independent living of the elderly by causing behavior disorders, gait disturbances, or falls. In addition, sarcopenia lowers the basal metabolic rate and increases insulin resistance, thereby promoting the development of type II diabetes, and increasing the risk of high blood pressure and cardiovascular disease 3- to 5-fold.

Currently, there are no drugs approved for the treatment of sarcopenia, and drug repositioning technology is under development to apply myostatin inhibitors or existing FDA-approved therapeutic agents for other diseases to the treatment of sarcopenia. In one example, Korean Patent No. 10-1666659 discloses that cetylpyridinium chloride, which has been used as a therapeutic agent for pharyngitis/stomatitis, has the effect of promoting myoblasts.

### [Disclosure]

### [Technical Problem]

The present invention is directed to providing a use of a 3-(4-(benzyloxy)phenyl)hex-4-inoic acid derivative for the prevention or treatment of muscle diseases.

### [Technical Solution]

To achieve the purpose, one aspect of the present invention provides a pharmaceutical composition for preventing or treating muscle diseases, which includes the compound of Formula 1, an optical isomer thereof, or a pharmaceutically acceptable salt thereof as an active ingredient.

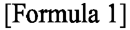

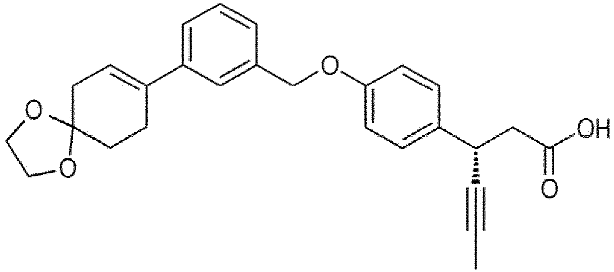

In the present invention, the muscle disease is a disease in which muscle mass is lost and which is one or more selected from the group consisting of sarcopenia, muscular atrophy, myasthenia gravis, muscular dystrophy, myotonia, hypotonia, and muscle weakness, and preferably, sarcopenia.

In the present invention, "sarcopenia" refers to a state in which physical activity is impaired by a decrease in skeletal muscle and muscle strength due to aging or a disease. Muscle loss generally begins in the 30s or 40s, and approximately half of the total muscle mass is lost by the 80s. The reduction in skeletal muscle, which is important metabolic tissue that can account for up to 60% of body mass, is an important cause of metabolic diseases such as insulin resistance, cardiovascular diseases, and falls.

Exercise, protein, and calorie supplementation are known to be helpful for sarcopenia but they do not much help in the elderly, who make up the majority of sarcopenia patients, so there is an urgent need for therapeutic agents for sarcopenia. However, drugs that exhibit a direct effect on improving muscle loss and increasing muscle mass are still at the clinical trial level, and there are currently no drugs that have been finally approved by the FDA.

In the present invention, "prevention" refers to all actions of inhibiting or delaying the onset of muscle loss-related diseases by administering the composition.

In the present invention, "treatment" refers to all actions involved in alleviating or beneficially changing symptoms of muscle loss-related diseases by administering the composition, such as the promotion of myogenesis, and a decrease in rate and amount of muscle loss.

The compound of Formula 1 according to the present invention is (3S)-3-(4-(3-(1,4-dioxaspiro[4,5]des-7-en-8-yl)benzyloxy)phenyl)hex-4-inoic acid, and the compound according to the present invention includes the compound of Formula 1 and a pharmaceutically acceptable salt thereof, as well as all solvates, optical isomers, and hydrates, which can be prepared therefrom.

When used in the form of a pharmaceutically acceptable salt, pharmaceutically acceptable metal salts may be prepared using bases. An alkali metal or alkaline earth metal salt is obtained by, for example, dissolving the compound in an excessive amount of alkali metal hydroxide or alkaline earth metal hydroxide solution, filtering out a non-dissolved compound salt, evaporating the filtrate, and drying it. Here, it is pharmaceutically suitable to prepare a sodium, potassium or calcium salt as a metal salt. In addition, the corresponding salt is obtained by reacting an alkali metal or alkaline earth metal salt with a suitable silver salt (e.g., silver nitrate).

Further, pharmaceutically acceptable salts may be prepared using an amino acid in which an amino group is attached to an organic acid, and as the amino acid salt, it is pharmaceutically suitable that, for example, natural amino acids such as glycine, alanine, phenylalanine, valine, lysine, and glutamic acid are used. Preferably, the pharmaceutically acceptable salt may be prepared with a lysine salt having the structure of Formula 2 below. Specifically, (3S)-3-(4-(3-(1,4-dioxaspiro[4,5]des-7-en-8-yl)benzyloxy)phenyl)hex-4-inoic acid and L-lysine salt may be dissolved in methanol, isopropyl acetate may be added, and then a lysine salt may be obtained from the reaction product.

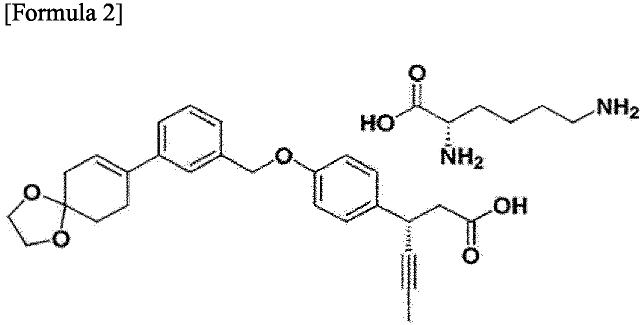

In addition, the lysine salt of Formula 2 is a crystalline form characterized as having an X-ray powder diffraction pattern with four or more diffraction peaks at 2[θ] values selected from 4.61 ± 0.2, 5.49 ± 0.2, 6.84 ± 0.2, 11.74 ± 0.2, 12.05 ± 0.2, 13.74 ± 0.2, 16.50± 0.2, 16.94 ± 0.2, 18.45 ± 0.2, 19.11 ± 0.2, 20.13 ± 0.2, 20.42 ± 0.2, 20.87 ± 0.2, 21.57 ± 0.2, 23.04 ± 0.2, and 25.02 ± 0.2.

Particularly, the X-ray powder diffraction pattern has a diffraction peak at a 2[0] value selected from 4.61 ± 0.2, 6.84 ± 0.2, 11.74 ± 0.2, 16.50± 0.2, 16.94 ± 0.2, 20.42 ± 0.2, and 20.87 ± 0.2.

More specifically, the crystalline form of the lysine salt of Formula 2 is characterized as having an X-ray powder diffraction pattern with peak positions matching the peak positions listed in Table 1 below.

**[Table 1]**

| Caption | Angle | d value | Intensity | Intensity % |
|---|---|---|---|---|
| | 2-Theta ° | Angstrom | Count | % |
| d=19.14506 | 4.612 | 19.14506 | 4735 | 89.4 |
| d=16.07776 | 5.492 | 16.07776 | 2226 | 42 |
| d=12.91070 | 6.841 | 12.9107 | 3738 | 70.6 |
| d=7.53027 | 11.743 | 7.53027 | 5231 | 98.8 |
| d=7.33717 | 12.053 | 7.33717 | 5296 | 100 |
| d=6.44222 | 13.735 | 6.44222 | 4485 | 84.7 |
| d=5.36804 | 16.501 | 5.36804 | 5215 | 98.5 |
| d=5.22939 | 16.941 | 5.22939 | 4947 | 93.4 |
| d=4.80573 | 18.447 | 4.80573 | 4289 | 81 |
| d=4.64015 | 19.112 | 4.64015 | 4100 | 77.4 |
| d=4.40719 | 20.132 | 4.40719 | 4188 | 79.1 |
| d=4.34614 | 20.418 | 4.34614 | 4519 | 85.3 |
| d=4.25406 | 20.865 | 4.25406 | 4692 | 88.6 |
| d=4.11754 | 21.565 | 4.11754 | 3688 | 69.6 |
| d=3.85747 | 23.038 | 3.85747 | 3021 | 57 |
| d=3.55665 | 25.016 | 3.55665 | 2478 | 46.8 |

The compound of Formula 1 is known to activate the G-protein coupled receptor (GPR)40 enzyme. GPR40 is a G-protein-coupled receptor (GPCR) that is mainly expressed in the insulin-secreting cells of the pancreas. The GPR40 expression profile has potential utility for the treatment of various metabolic diseases, including obesity and diabetes. However, the myogenesis promoting or muscle loss inhibiting effect of the compound of Formula 1 is not known yet.

As a result of treating C2C12 cells, which are myoblasts, with the compound of Formula 1 to determine whether the compound of Formula 1 affects muscle loss or myogenesis, the present inventors confirmed that the expression of myogenic factors MyoG and MyHC increases, and myotube formation is promoted (Experimental Example 1).

In addition, the compound of Formula 1 may inhibit muscle loss even in a situation in which sarcopenia is accelerated such as diabetes, obesity, and inflammatory responses. Specifically, when C2C12 cells are treated only with palmitate, a Bax/Bcl2 ratio increases, cell viability decreases, the expression of MyoG and MyHC decreases, and the MyHC positive area and myotube thickness also decrease, resulting in a muscle loss phenomenon. However, when C2C12 cells are treated with both palmitate and the compound of Formula 1, such a phenomenon is reversed, indicating that muscle loss is inhibited by the compound of Formula 1 (Experimental Example 2).

Likewise, the compound of Formula 1 exhibits excellent myogenesis promoting and muscle loss inhibiting effects. Therefore, in addition to providing the pharmaceutical composition for preventing or treating muscle diseases, the present invention also provides a use of the compound of Formula 1, or an optical isomer or pharmaceutically acceptable salt thereof for preparing the pharmaceutical composition for preventing or treating muscle diseases, and a method of preventing or treating muscle diseases, which includes administering a therapeutically effective amount of the compound of Formula 1, or an optical isomer or pharmaceutically acceptable salt thereof to a subject.

In the present invention, "subject" refers to a target in need of treatment for a disease, and more specifically, a mammal such as a human or a non-human primate, a mouse, a rat, a dog, a cat, a horse, or a cow, which is in need of treatment for muscle diseases.

The pharmaceutical composition of the present invention may be administered orally or parenterally (e.g., intravenously, subcutaneously, intraperitoneally, or locally) according to a desired method, and when formulated, is prepared using a diluent or excipient, such as a filler, an extender, a binder, a wetting agent, a disintegrant, or a surfactant, which is normally used.

A solid formulation for oral administration may be a tablet, a pill, a powder, a granule, a capsule, or a troche, and is prepared by adding one or more excipients, for example, starch, calcium carbonate, sucrose or lactose, or gelatin, to one or more of the compounds of the present invention. In addition, other than such simple excipients, lubricants such as magnesium stearate and talc are also used. As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included.

A formulation for parenteral administration may be a sterilized aqueous solution, a non-aqueous solvent, a suspension, an emulsion, a lyophilized preparation or a suppository. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurinum, glycerol, or gelatin may be used.

In addition, the effective amount of the pharmaceutical composition of the present invention may vary depending on a patient's age, sex, condition and body weight, an absorption rate of the active ingredient in the body, an inactivation rate, an excretion rate, the type of disease, or a drug used in combination, and the compound of Formula 1 may be generally administered, but not limited to, at 0.001 to 200 mg, preferably, 0.01 to 100 mg, and more preferably, 0.2 to 50 mg per kg of body weight when administered once to several times a day. However, the effective amount may vary depending on an administration route, the severity of obesity, sex, body weight or age, and therefore, the scope of the present invention is not limited by the dose in any way.

Another aspect of the present invention provides a method of treating muscle diseases, which includes administering a pharmaceutical composition for preventing or treating muscle diseases to a subject in need of treatment.

Among the terms or elements mentioned in the method of treating muscle diseases, those mentioned in the description of the pharmaceutical composition for preventing or treating muscle diseases are understood to be the same as mentioned above in the description of the composition.

### [Advantageous Effects]

The 3-(4-(benzyloxy)phenyl)hex-4-inoic acid derivative of the present invention can promote the expression of myogenic factors, MyoG and MyHC, inhibit the expression of a muscular atrophy factor, atrogin-1, promote the formation of myotubes, and inhibit myocyte apoptosis, resulting in the promotion of myogenesis and inhibition of muscle loss.

### [Description of Drawings]

FIG. 1 is a result of confirming the change in expression of MyoG and MyHC after treating myoblasts, C2C12 cells, with Compound A.
FIG. 2 is a result of confirming the changes in MyHC positive area and myotube thickness after treating C2C12 cells with Compound A.
FIG. 3 is a result of confirming the changes in Bax/Bcl2 ratio and cell viability according to the presence or absence of Compound A treatment after treating C2C12 cells with palmitate.
FIG. 4 is a result of confirming the changes in the expression of MyoG and MyHC according to the presence or absence of Compound A treatment after treating C2C12 cells with palmitate.
FIG. 5 is a result of confirming the changes in MyHC positive area and myotube thickness according to the presence or absence of Compound A treatment after treating C2C12 cells with palmitate.
FIG. 6 is a result of confirming the change in atrogin-1 expression according to the presence or absence of Compound A treatment after treating C2C12 cells with palmitate.
FIG. 7 is a result of confirming the change in MyoG and MyHC expression after treating C2C12 cells with a GPR40 agonist LY2922470.
FIG. 8 is a result of confirming the change in MyHC positive area after treating C2C12 cells with LY2922470.
FIG. 9 is a result that confirms the change in cell viability according to the presence or absence of LY2922470 treatment after treating C2C12 cells with palmitate.

### [Detailed Description of Exemplary Embodiments]

Hereinafter, one or more embodiments will be described in further detail with reference to examples. However, these examples are provided to illustrate one or more embodiments, and the scope of the present invention is not limited to the following examples.

### Experimental Example 1: Myogenesis promoting effect of Compound A

T compound used in the present invention is a lysine salt, which is (3S)-3-(4-(3-(1,4-dioxaspiro[4,5]des-7-en-8-yl)benzyloxy)phenyl)hex-4-inoic acid, (hereinafter, referred to as Compound A).

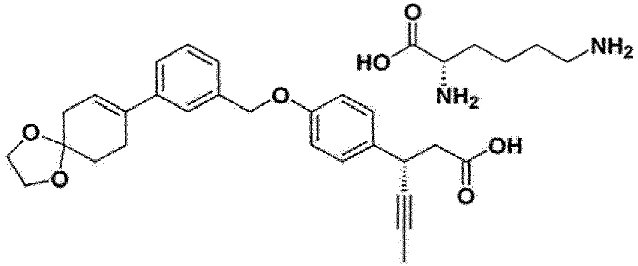

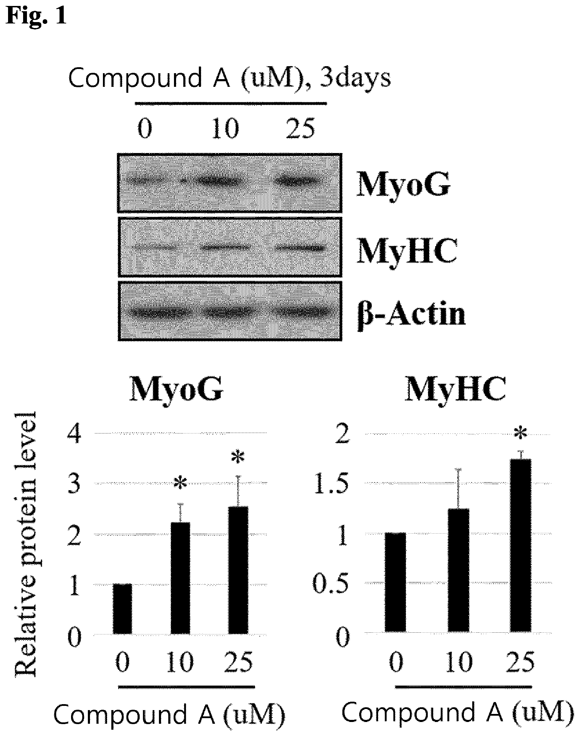

C2C12 cells, which are myoblasts, were cultured and treated with a mixture of Compound A in a differentiation medium [2% horse serum (Cat No. 16050, Invitrogen, USA)-containing DMEM (Cat No. 11965, Invitrogen)] for 3 days. Afterward, the change in expression of myogenin (myogenic factor 4, MyoG) and a myosin heavy chain (MyHC), which are known as myogenic factors, was confirmed by western blotting. As primary antibodies, a MyoG antibody (Santa Cruz, sc-12732), a MyHC antibody (Santa Cruz, sc-32732), and a beta-actin antibody (Santa Cruz, sc-47778) were used. As a result, it can be confirmed that the higher the treatment concentration of Compound A, the higher the expression of MyoG and MyHC (FIG. 1).

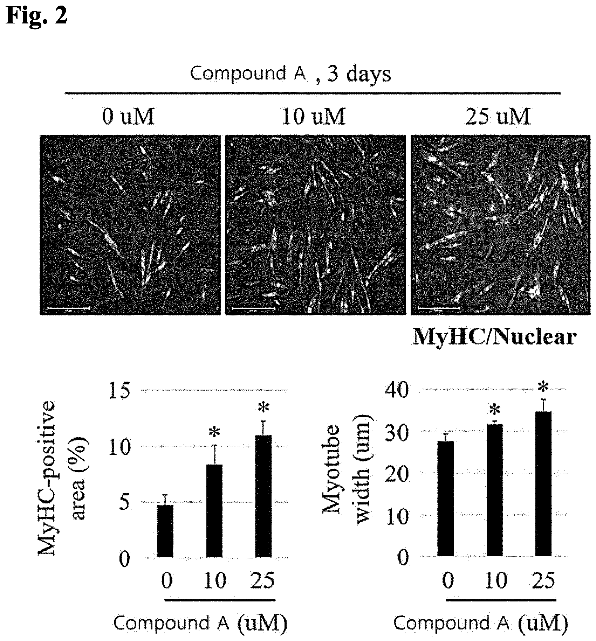

In addition, C2C12 cells were treated with Compound A in the same manner as above, and then immunohistochemistry was performed with a MyHC antibody. As a result, it was confirmed that, as the treatment concentration of Compound A increases, the MyHC positive area and myotube thickness increase (FIG. 2).

From the results shown in FIGS. 1 and 2, it can be seen that Compound A is involved in myogenesis promotion.

### Experimental Example 2: Muscle loss inhibition effect of Compound A

Sarcopenia is known to be accelerated by diabetes, obesity, or inflammatory responses. Accordingly, it was confirmed whether Compound A can inhibit muscle loss even under the above conditions as follows. Palmitate (PA; P9767, Sigma-Aldrich, USA) was dissolved in ethanol and then mixed with bovine serum albumin (BSA; A7888, Sigma-Aldrich) dissolved in DMEM at a molar ratio of 6:1, followed by conjugation at 60 °C for 1 hour. Cells were treated with the resulting conjugate.

After mixing Compound A and the palmitate in a growth medium (10% FBS (Cat No. 16000, Invitrogen)-containing DMEM), C2C12 cells were treated with the resulting mixture for 24 hours, and then a Bax/Bcl2 ratio was confirmed by western blotting and cell viability was assessed using a cell viability assay kit (Dozen, EZ-3000). Primary antibodies used herein were the Bax antibody (Santa Cruz, sc-7480) and Bcl2 antibody (Santa Cruz, sc-7382). Bax is a protein inducing apoptosis, and Bcl2 is a protein inhibiting apoptosis by hindering the migration of Bax to mitochondria.

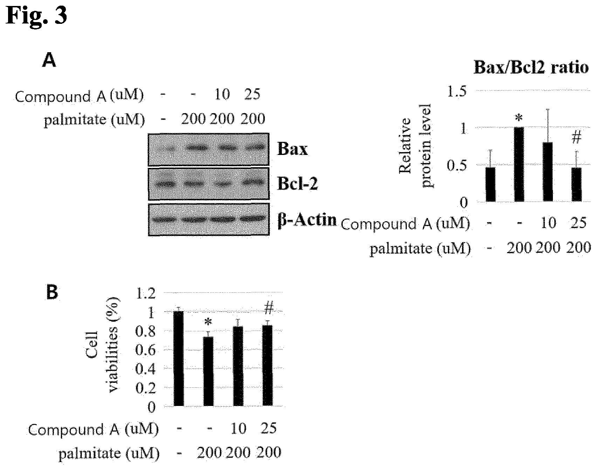

As a result, it was confirmed that the Bax/Bcl2 ratio increased and cell viability decreased when C2C12 cells were treated with only palmitate, whereas the Bax/Bcl2 ratio decreased and cell viability increased when both palmitate and Compound A were treated (FIG. 3).

Compound A and the palmitate were mixed in a growth medium (10% FBS-containing DMEM), and C2C12 cells were treated with the resulting mixture. After 24 hours, the medium was exchanged with a fresh growth medium to induce differentiation for 3 days, and after 3 days, the cells were collected to confirm the expression of MyoG and MyHC, and the changes in MyHC positive area and myotube thickness.

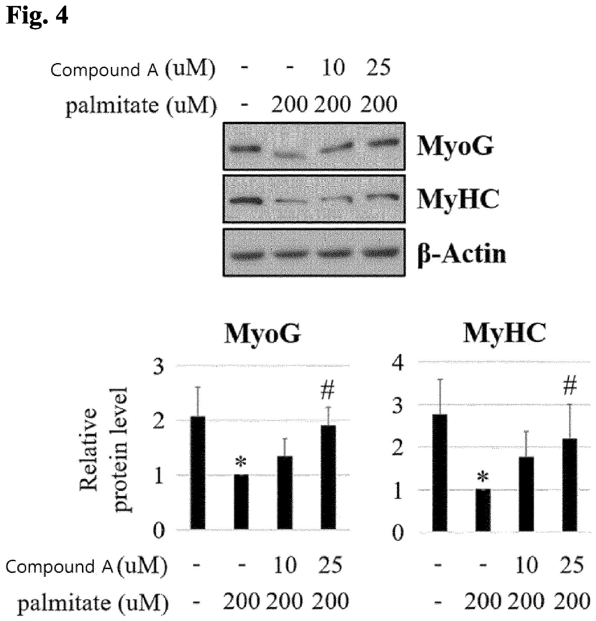

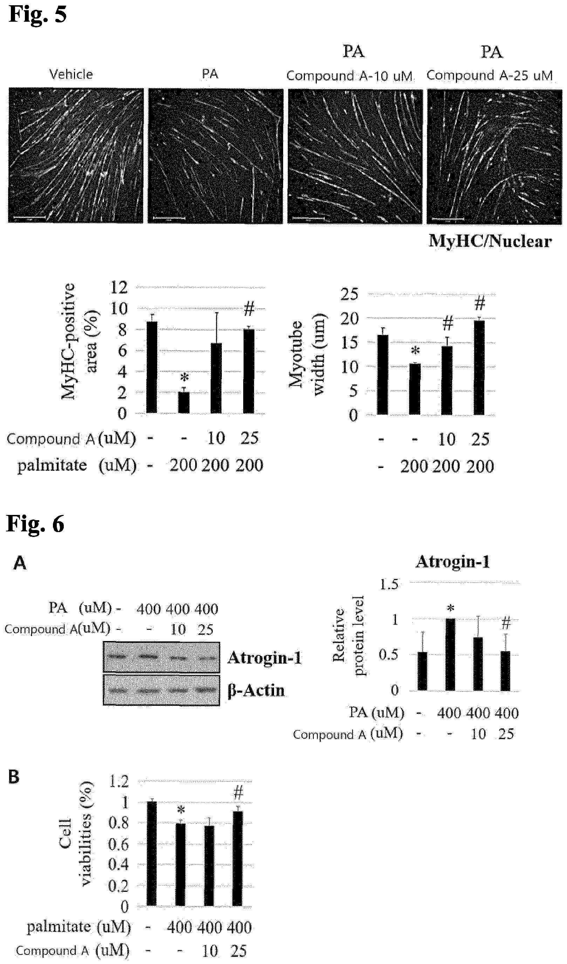

As a result, it can be seen that, when the C2C12 cells were treated with only palmitate, the expression of MyoG and MyHC, the MyHC positive area, and the myotube thickness decreased, but when both palmitate and Compound A were treated, the above results were reversed (FIGS. 4 and 5).

Atrogin-1 is a type of E3 ubiquitin ligase and a marker associated with muscular atrophy, and it is known that, as its gene expression increases, muscle is broken down. To confirm the change in atrogin-1 expression by Compound A, C2C12 cells were cultured in a differentiation medium for 6 days to induce differentiation into myotubes, and the differentiated cells were treated with a mixture of Compound A and palmitate in a differentiation medium. Afterward, an expression level was confirmed using an atrogin-1 antibody (Abnova, PAB15627), and cell viability was measured.

As a result, it was confirmed that, when the C2C12 cells were treated with only palmitate, atrogin-1 expression increased, but when both palmitate and Compound A were treated, atrogin-1 expression decreased (FIG. 6A). In addition, it can be seen that the cell viability decreased by palmitate treatment was recovered with Compound A treatment (FIG. 6B).

### Experimental Example 3: Confirmation of myogenesis promoting effect of LY2922470

Since Compound A is known as a G-protein-coupled receptor40 agonist (GPR40 agonist), it was confirmed whether the myogenesis promoting effect (Experimental Example 1) of Compound A is a general effect of GPR40 agonists.

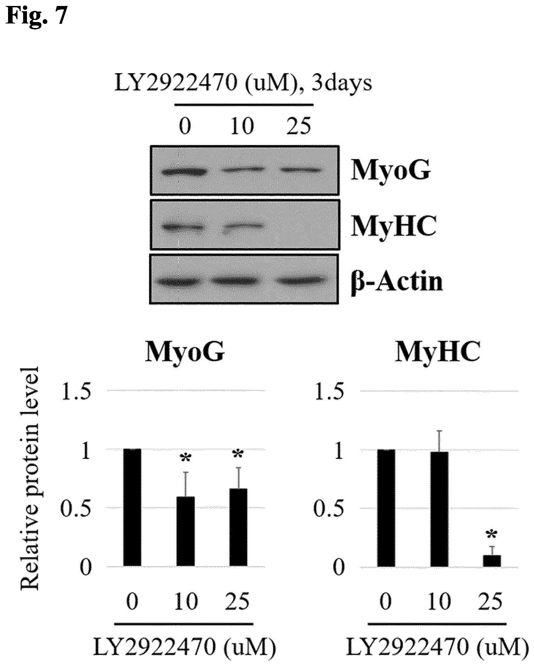

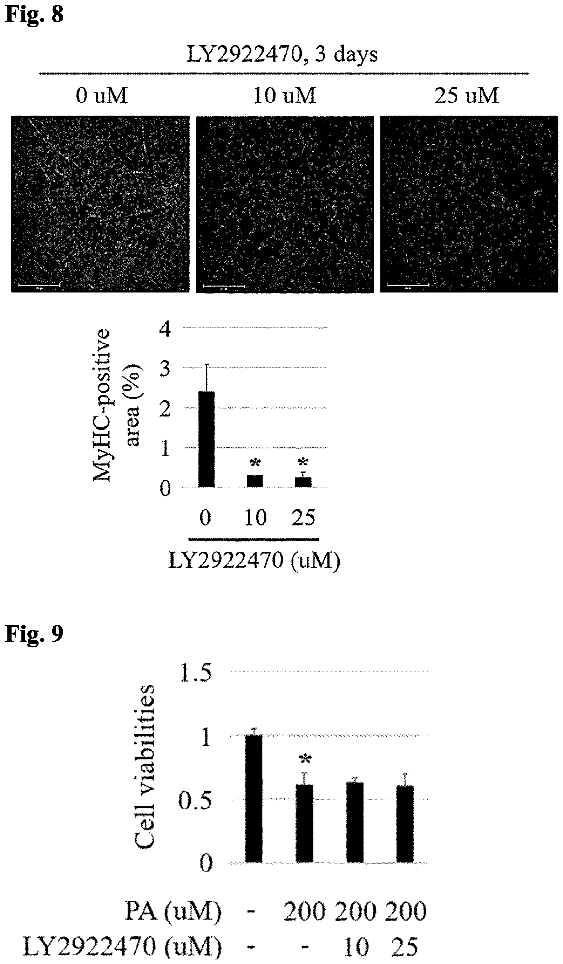

Specifically, experiments were conducted in the same manner as in Experimental Examples 1 and 2, except that C2C12 cells were treated with LY2922470 instead of Compound A. As a result of the experiments, it can be seen that, when the C2C 12 cells were treated with LY2922470, the expression of myogenic factors MyoG and MyHC was inhibited (FIG. 7), the MyHC positive area decreased, and myotube formation was inhibited (FIG. 8).

In addition, although it was confirmed that Compound A inhibited apoptosis caused by palmitate treatment (FIG. 3B), LY2922470 did not inhibit apoptosis caused by palmitate (FIG. 9).

It is expected that the difference in effects between Compound A and LY2922470 is due to a biased agonism depending on the GPR40 ligand, and from the results of this Experimental Example, it can be seen that not all GPR40 agonists have a myogenesis promoting effect.

## Claims

1. A pharmaceutical composition for use in preventing or treating muscle diseases, comprising:
a compound of Formula 1, an optical isomer thereof, or a pharmaceutically acceptable salt thereof as an active ingredient,
wherein the muscle disease includes one or more diseases selected from the group consisting of sarcopenia, muscular atrophy, myasthenia gravis, muscular dystrophy, myotonia, hypotonia, and muscle weakness.

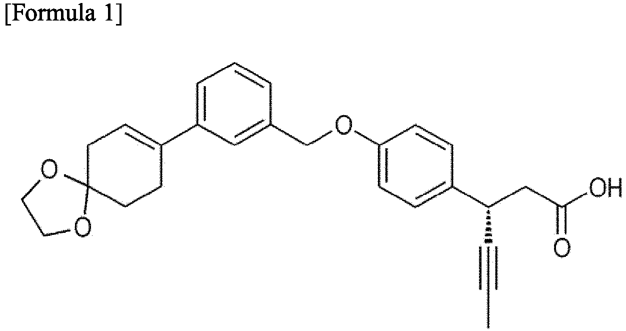

2. The pharmaceutical composition of claim 1, wherein the compound of Formula 1 promotes the expression of a myogenic factor, myogenin (MyoG) or a myosin heavy chain (MyHC).

3. The pharmaceutical composition of claim 1, wherein the compound of Formula 1 reduces the expression of a muscular atrophy factor, atrogin-1.

4. The pharmaceutical composition of claim 1, wherein the pharmaceutically acceptable salt of the compound of Formula 1 is a lysine salt having the structure of Formula 2 below.

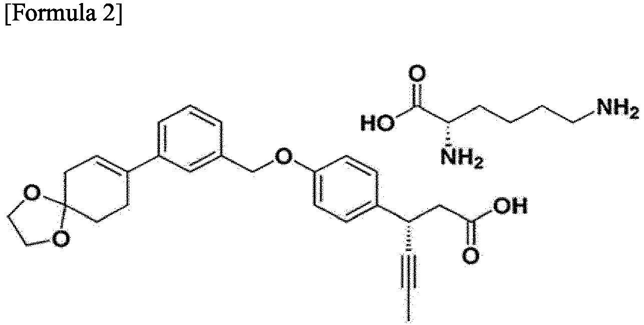

5. The pharmaceutical composition of claim 4, wherein the lysine salt having the structure of Formula 2 is characterized as having an X-ray powder diffraction pattern with four or more diffraction peaks at 2[θ] values selected from 4.61 ± 0.2, 5.49 ± 0.2, 6.84 ± 0.2, 11.74 ± 0.2, 12.05 ± 0.2, 13.74 ± 0.2, 16.50± 0.2, 16.94 ± 0.2, 18.45 ± 0.2, 19.11 ± 0.2, 20.13 ± 0.2, 20.42 ± 0.2, 20.87 ± 0.2, 21.57 ± 0.2, 23.04 ± 0.2, and 25.02 ± 0.2.

6. A method of treating muscle diseases, comprising:
administering the pharmaceutical composition for preventing or treating muscle diseases according to any one of claims 1 to 5 to a subject in need of treatment,
wherein the muscle disease includes one or more diseases selected from the group consisting of sarcopenia, muscular atrophy, myasthenia gravis, muscular dystrophy, myotonia, hypotonia, and muscle weakness.
